Europäisches Patentamt

European Patent Office

Office européen des brevets

(19)

(11) Veröffentlichungsnummer: **0 105 111 B1**

(12) ## EUROPÄISCHE PATENTSCHRIFT

(45) Veröffentlichungstag der Patentschrift:
17.04.85

(51) Int. Cl.⁴: **C 07 C 69/14,** C 07 C 67/08

(21) Anmeldenummer: **83107249.1**

(22) Anmeldetag: **23.07.83**

(54) Verfahren zur Veresterung von Essigsäure mit Alkoholen, die 4 und mehr C-Atome enthalten, sowie mit Glykolethern.

(30) Priorität: **25.09.82 DE 3235531**

(43) Veröffentlichungstag der Anmeldung:
**11.04.84 Patentblatt 84/15**

(45) Bekanntmachung des Hinweises auf die Patenterteilung:
**17.04.85 Patentblatt 85/16**

(84) Benannte Vertragsstaaten:
**BE DE FR GB IT NL SE**

(56) Entgegenhaltungen:
**DE - A - 3 121 383**
**DE - B - 1 919 527**
**US - A - 4 314 947**

(73) Patentinhaber: **CHEMISCHE WERKE HÜLS AG, - RSP Patente / PB 15 - Postfach 13 20, D-4370 Marl 1 (DE)**

(72) Erfinder: **Alfs, Helmut, Bitterfelder Strasse 47, D-4370 Marl (DE)**
Erfinder: **Böxkes, Werner, Dr., Oppauer Strasse 2, D-4370 Marl (DE)**
Erfinder: **Vangermain, Dr., Leverkusener Strasse 1, D-4370 Marl (DE)**

## Beschreibung

Es ist bekannt, Essigsäureester technisch durch Veresterung der Essigsäure mit Alkoholen in flüssiger Phase herzustellen.

Die wichtigsten Katalysatoren für die Veresterung von Essigsäure mit Alkoholen in der Flüssigphase sind Brönsted-Säuren, insbesondere Schwefelsäure.

Außer Schwefelsäure sind auch Chlorsulfonsäuren, Toluolsulfosäuren sowie Gemische von Schwefelsäure mit organischen, beispielsweise p-Toluolsulfonsäure, oder schwächeren anorganischen Säuren, wie Phosphorsäure, geeignet.

So kann man z. B. Essigsäureester nach dem Verfahren der FR-PS 1 068 367 und der DE-PS 824 341 in Gegenwart von Schwefelsäure herstellen. Veresterungen in Gegenwart von im Reaktionsgemisch homogenen gelösten Säurekatalysatoren haben aber den Nachteil, daß sie in Apparaturen aus kostspieligen, korrosionsbeständigen Materialien, vorwiegend emaillierten Anlagenteilen, durchgeführt werden müssen. Außerdem muß ein Teil der durch Veresterung verbrauchten Säure (z. B. Schwefelsäure oder Cumolsulfosäure) aus dem Prozeß abgezogen werden. Die abgezogenen Anteile verbrauchter Säure müssen vor Ablauf in das Fabrikationswasser neutralisiert werden und stellen eine nicht unerhebliche Umweltbelastung dar.

Man kann zwar nach der DE-PS 975 700 beispielsweise Monoglykolether in Gegenwart von einem Gemisch aus Toluolsulfosäure und Phosphorsäure als Katalysator mit Essigsäure verestern und dadurch die Korrosivität der Reaktionsmischung erniedrigen, die übrigen Nachteile aber nicht eliminieren. Diese Nachteile lassen sich durch Einsatz von sauren Ionenaustauschern, insbesondere auf Basis von mit Divinylbenzol vernetzten sulfosauren Vinylpolymeren vermeiden (Chem. Technik 5 (1953) 187; Chem. Technik 11 (1959) 24 ff.; Angew. Chemie 66 (1954) 241).

Diese Veresterungen haben aber große Nachteile, wie in Chemie-Technik, 11 (1959) Seiten 24 bis 26 beschrieben. Neben den geringen Durchsätzen wegen der Raum-Zeit-Ausbeuten von nur 0,2 bis 1,07 kg Butylacetat/kg Katalysator muß der Sumpfinhalt — um Säure- und Alkoholfreiheit zu erhalten — auch noch bis zum Siedepunkt des gebildeten Esters erhitzt werden. Die dabei in die mit Ionenaustauschern gefüllte Veresterungskolonne zwangsläufig emporsteigenden Esterdämpfe werden hierbei in erheblichem Maße gespalten. So zersetzt sich beispielsweise das wasserfreie Acetat des Monoglykolethers schon bei 110°C in Gegenwart von stark sauren Ionenaustauschern erheblich, u. a. in Ethylacetat und Ethyldiglykoletheracetat. Außerdem unterliegen die hier eingesetzten, grobstückigen Katalysatoren starkem Abrieb und haben dadurch kurze Standzeiten.

Dieses Verfahren des Standes der Technik betreffen nur die Herstellung von Essigsäureestern mit niedermolekularen Alkoholen mit 1 bis 4 Kohlenstoffatomen. Technische Verfahren zur Veresterung von Essigsäure mit höheren Akoholen und Glykolethern in Gegenwart von sauren Ionenaustauschern sind bisher nicht bekannt.

Daraus ergibt sich die Aufgabe, ein Verfahren zu finden, das die Nachteile des Standes Technik vermeidet und auch die Herstellung von Essigsäureestern mit höheren Alkoholen und Glykolethern in einfacher und wirtschaftlicher Weise in guter Ausbeute bei hohen Raum-Zeit-Ausbeuten ermöglicht.

Diese Aufgabe wird erfindungsgemäß entsprechend den Angaben der Patentansprüche gelöst.

Das erfindungsgemäße Verfahren eignet sich für die Veresterung von Essigsäure mit Alkoholen, die 4 und mehr C-Atome haben, wie n-, iso- und tert.-Butanol, Amylalkohole, Hexanole, Heptanole, Oktanole, beispielsweise 2-Ethylhexanol und Glykolether, wie Ethylmonoglykolether, Ethyldiglykolether.

Das Molverhältnis Alkohol zu Essigsäure beträgt im allgemeinen 0,5 bis 10 : 1, vorzugsweise 1,0 bis 2 : 1.

Die Veresterung führt man im allgemeinen bei einer Temperatur von 70 bis 140°C durch, vorzugsweise bei einer Temperatur von 90 bis 120°C, in einem Überdruckbereich von 1,01 bis 10 bar. Den Druck im Reaktor regelt man mit Hilfe eines Drucküberstromventils, so daß der Katalysator mit der Reaktionsmischung geflutet ist.

Als Katalysatoren sind grundsätzlich alle stark sauren Kationenaustauscher geeignet. Sie können sowohl gelförmig als auch makroporös sein. Im allgemeinen setzt man handelsübliche Ionenaustauscher, beispielsweise mit Divinylbenzol vernetzte Polystyrolsulfosäure, mit einem Kornspektrum von 0,4 bis 1,3 mm ein. Ein Teil der $H^+$-Ionen der sauren Kationenaustauscher kann durch Metalle ersetzt sein. Die Katalysatormenge ist vorteilhaft so zu bemessen, daß das ablaufende Gemisch sich ins Gleichgewicht gesetzt hat, entsprechend einem Durchsatz von 1 bis 20 g des Einsatzgemisches/h/g trockenen Kontaktes. Vor dem Einsatz läßt man den Kationenaustauscher in dem umzusetzenden Alkohol bis zur Volumenkonstanz quellen.

Als Veresterungsreaktoren eignen sich alle vertikalen Reaktortypen, die üblicherweise als Festbettreaktoren eingesetzt werden, wie Schachtöfen oder Rohrbündelreaktoren; vorteilhaft ist ein Schachtofen, der am unteren Ende vor der Verjüngung einen Flansch besitzt. Zwischen dem Flansch befindet sich auf einer Lochblechplatte ein feinmaschiges Sieb mit einer Maschenweite von beispielsweise 0,5 mm. Die Veresterung führt man in dem vertikalen Reaktor im Ab- oder Aufstrom, vorteilhaft im Abstrom, durch und entspannt das Veresterungsgemisch mit Hilfe eines Drucküberstromventils in eine nachgeschaltete Destillationskolonne. Das bei der Veresterung gebildete Wasser destilliert man mit einem Schleppmittel aceotrop über Kopf ab. Als Schleppmittel sind beispielsweise Benzol oder Toluol geeignet.

Die Veresterung führt man beispielsweise in der Weise durch (s. Abb.), daß man eine Mischung aus Essigsäure und einem Alkohol mit mindestens 4 C-Atomen bzw. einem Glykolether aus einem Dosiergefäß (1) über eine Vorlage (2) mit einer Dosierpumpe (3) in einem Wärmetauscher (4) fördert und dort auf die Reaktionstemperatur von 70 bis 140° C aufheizt. Diese aufgeheizte Mischung gibt man von oben in einen vertikalen Reaktor (5), in dem sich der stark saure Kationenaustauscher befindet, der zuvor in dem Alkohol bis zur Volumenkonstanz gequollen ist.

Der Reaktor wird durch einen Wärmeträger auf Reaktionstemperatur gehalten. Im Reaktor erfolgt die Veresterung bis zum Gleichgewicht. Mit Hilfe eines Drucküberstromventils (12) hält man den Druck im Reaktor so hoch, daß der Reaktor geflutet betrieben wird. Das den Reaktor unten verlassende Veresterungsgemisch entspannt man über das Drucküberstromventil in die obere Hälfte der nachgeschalteten Destillationskolonne (6). Das Reaktionswasser destilliert man mit einem Schleppmittel, beispielsweise Benzol oder Toluol aceotrop über Kopf ab. Das Wasser trennt man in einem Phasenabscheider (7) als untere Schicht (8) ab und fährt das Schleppmittel in die Kolonne zurück.

Den gebildeten Ester zieht man dampfförmig unterhalb des ersten Kolonnenbodens ab (9). Hochsiedende Verunreinigung schleust man flüssig aus der Blase (10) nach (11) aus. Den erhaltenen Rohester kann man anschließend in bekannter Weise in hintereinandergeschalteten Kolonnen zum Reinester aufarbeiten. Die nicht umgesetzten Einsatzstoffe zieht man vorteilhafterweise kontinuierlich als Seitenstrom aus dem Abtriebsteil der Kolonne ab, kondensiert und führt in die Vorlage (2) zurück. Aus diesem Rückstrom können ggf. geringe Mengen leichtsiedende Nebenprodukte wie z. B. Ethylacetat (kann bei der Veresterung von Ethylmonoglykolether entstehen) destillativ vorher entfernt werden.

Nach Ergänzung mit frischem Alkohol-Essigsäure-Gemisch aus (1) in einer dem aus dem Prozeß abgezogenen Ester entsprechenden Menge führt man das gesamte Gemisch nach Erwärmen in (4) wieder dem Reaktor zu. Selbstverständlich kann man die nicht ungesetzten Einsatzprodukte auch mit dem Rohester aus der Blase abziehen und mit Hilfe von Aufarbeitungskolonnen die Einsatzstoffe eliminieren und dem Prozeß wieder zuführen.

Die erfindungsgemäß erhaltenen Essigsäureester verwendet man als Lösemittel für Alkyl- und Vinylharze, Nitrocellulose, Celluloid, Chlorkautschuk und als Lacklösemittel.

## Beispiel 1 (siehe Abb.)

### Ethylmonoglykoletheracetat

In ein ummanteltes Rohr (5) (Ø 35 mm, Höhe 65 cm) werden 150 g trockener, bis zur Volumenkonstanz mit Ethylmonoglykolether gequollener, stark saurer Kationenaustauscher (Polystyrolsolfonsäure mit 18 Gew.-% Divinylbenzol vernetzt, Korngröße 0,3 bis 1,3 mm) eingefüllt. Das Volumen des gequollenen Katalysators beträgt 480 m$^3$.

Das Katalysatorbett ruht auf einem VA-Maschengewebe von 0,3 mm Maschenabstand, so daß ein Austragen des Katalysators während der Veresterung aus dem Reaktor verhindert wird. 600 g/h eines Gemisches Ethylmonoglykolether — Essigsäure im Molverhältnis 1,1 : 1 aus der Vorlage (1) fördert man mittels Dosierpumpe (3) durch den Vorwärmer (4) und heizt auf 115° C auf.

Aus dem Vorwärmer durchströmt das Gemisch von oben nach unten den Reaktor (5). Die Reaktionstemperatur im Reaktor beträgt 115° C. Mit Hilfe des Drucküberstromventils (12) stellt man im Reaktor einen Druck von 2 bar ein. Die Temperatursteuerung erfolgt durch Umpumpen von therostatisch gesteuertem Glykol durch die Heizmäntel des Vorwärmers und des Reaktors. Das den Reaktor verlassende flüssige Veresterungsgemisch wird über das Drucküberstromventil (12) in die obere Hälfte der dem Reaktor nachgeschalteten Füllkörperkolonne (6) (Ø 50 mm, Länge 180 cm, Füllkörper 6 mm Ringe) eingefahren, deren Sumpftemperatur 158 bis 160° C beträgt. Das Reaktionswasser destilliert man mit Toluol als Schleppmittel bei einer Temperatur von 80° C aceotrop über Kopf. Das Wasser wird in einem Phasenabscheider (7) als untere Schicht im äquimolaren Verhältnis abgezogen, während das Schleppmittel in die Kolonne zurückgefahren wird. Der gebildete Ester wird dampfförmig (9) bei 155 bis 160° C unterhalb des ersten Kolonnenbodens abgezogen, wobei hochsiedende Verunreinigungen aus dem Flüssigteil der Blase ausgeschleust werden (11). Den Roh-Ester arbeitet man in bekannter Weise in hintereinander geschalteten Kolonnen auf.

Es werden stündlich 520 g Rohester aus dem Dampfraum (9) unterhalb des ersten Kolonnenbodens abgezogen.

Der erhaltene Rohester hat folgende Zusammensetzung (ermittelt durch GC-Analyse (wasserfrei gerechnet):

| | |
|---|---|
| Ethylglykol: | 5,2 bis 6,0 Gew.-% |
| Ethylglykol-monoglykoletheracetat: | 93 bis 94 Gew.-% |
| unbekannte Hochsieder: | 0,8 Gew.-% |
| Raum-Zeit-Ausbeute | 3,25 kg Ester/h/kg Katalysator |

3

Die Ausbeute an Ethylmonoethyletheracetat, bezogen auf die umgesetzten Komponenten Alkohol-Essigsäure beträgt 97% d. Th.

Beispiel 2

Ethylhexylacetat

600 g/h einer Mischung 2-Ethylhexanol/Essigsäure im Verhältnis 1,2 Mol Ethylhexanol : 1,0 Mol Essigsäure setzt man nach den Angaben des Beispiels 1 um. Die Temperatur im Reaktor beträgt 110°C bis 115°C.

Stündlich werden bei einer Blasentemperatur von 200°C (Kolonne 6) 518 g Rohester aus dem Dampfraum (9) unterhalb des ersten Kolonnenbodens abgezogen. Der Rohester hat gaschromatographisch bestimmt (wasserfrei gerechnet) folgende Zusammensetzung:

| | |
|---|---|
| Ethylhexanol: | 11,5 Gew.-% |
| Ethylhexylacetat: | 88,3 Gew.-% |
| unbekannte Anteile | 0,2 Gew.-% |
| Raum-Zeit-Ausbeute | 3,05 kg Ester/h/kg Katalysator |

Die Ausbeute an Ethylhexylacetat, bezogen auf die eingesetzten Komponenten beträgt (unter Berücksichtigung der Apparatefüllung) 98% d. Th.

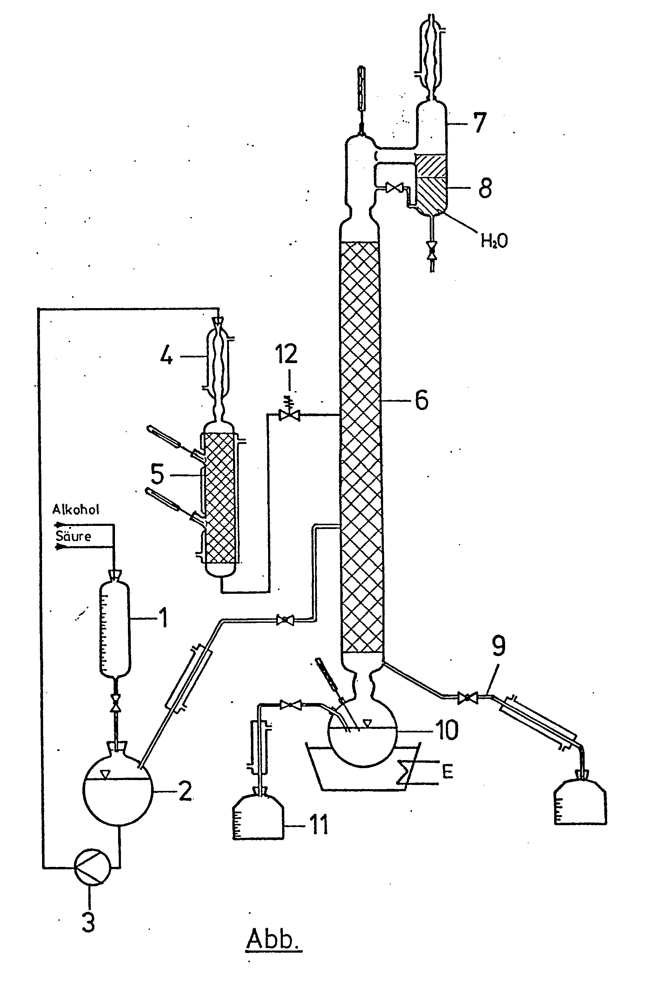

**Patentansprüche:**

1. Verfahren zur Veresterung von Essigsäure mit Alkoholen, die 4 und mehr C-Atome enthalten, sowie mit Glykolethern in Gegenwart von feinkörnigen, stark sauren Ionenaustauscher in vertikalen Reaktoren und destillativer Trennung des erhaltenen Reaktionsgemisches, dadurch gekennzeichnet, daß man die Veresterung in einem vertikalen Reaktor im Ab- oder Aufstrom durchführt, das den Reaktor verlassende Reaktionsgemisch in die obere Hälfte einer nachgeschalteten Destillationskolonne einführt, wobei man mit Hilfe eines Drucküberstromventils zwischen Reaktor und Destillationskolonne den Reaktor geflutet betreibt, über Kopf der Kolonne das Wasser mit einem Schleppmittel aceotrop austrägt, den gebildeten Ester dampfförmig unterhalb des ersten Kolonnenbodens abzieht, die nicht umgesetzten Anteile Essigsäure und Alkohol nach destillativer Abtrennung und nach Ergänzung mit Frischessigsäure-Alkohol — entsprechend der Menge des unterhalb der ersten Kolonnenbodens abgezogenen Esters — wieder dem Veresterungsreaktor zuführt und die gebildeten Hochsieder kontinuierlich flüssig aus dem Blasensumpf abzieht.

2. Verfahren nach Anspruch 1, dadurch gekennzeichnet, daß man die nicht umgesetzten Anteile Essigsäure und Alkohol aus dem Abtriebsteil der Destillationskolonne im Seitenstrom abzieht.

**Claims**

1. A process for esterifying acetic acid with an alcohol of 4 or more carbon atoms or with a glycol ether in the presence of a finely divided, strongly acidic ion exchanger in a vertical reactor with distillative separation of the resulting reaction mixture, characterised in that the esterification is carried out in a vertical reactor with descending or ascending flow, the reaction mixture leaving the reactor is introduced into the upper half of a downstream distillation column, the reactor is operated flooded with the aid of a pressure overflow valve between the reactor and distillation column, the water is removed azeotropically with an entraining agent via the top of the column, the ester formed is taken off as vapour below the first column tray, the unconverted amounts of acetic acid and alcohol are recycled to the esterification reactor after having been separated off by distillation and replenished with fresh quantities of acetic acid and alcohol corresponding to the amount of the ester taken off below the first column tray, and the high-boiling products formed are continuously taken off as liquid from the base of the column.

2. A process according to claim 1, characterised in that the unconverted amounts of acetic acid and alcohol are taken off as a side stream from the stripping section of the distillation column.

**Revendications**

1. Procédé d'estérification de l'acide acétique avec des alcools qui contiennent 4 atomes de carbone et davantage, ainsi qu'avec des éthers de glycol en présence d'échangeurs d'ions fortement acides à

**0 105 111**

grain fin, dans des réacteurs verticaux, et de séparation par distillation du mélange réactionnel obtenu, caractérisé par le fait que l'on effectue l'estérification dans un réacteur vertical, en courant descendant ou ascendant, que l'on introduit le mélange réactionnel quittant le réacteur dans la moitié supérieure d'une colonne de distillation montée en aval, en faisant fonctionner le réacteur à l'état noyé à l'aide d'une valve de décharge de pression entre réacteur et colonne de distillation, que par la tête de la colonne on évacue l'eau par voie azéotropique avec un entraîneur, que l'on retire l'ester formé, sous forme de vapeur, en-dessous du premier plateau de la colonne, que l'on renvoie dans le réacteur d'estérification les fractions d'acide acétique et d'alcool qui n'ont pas réagi, après séparation par distillation et après complément d'acide acétique et d'alcool frais — en fontion de la quantité de l'ester soutiré en-dessous du premier plateau de la colonne — et que l'on retire de façon continue du bas du réacteur, à l'état liquide les corps à point d'ébullition élevé formés.

2. Procédé selon la revendication 1, caractérisé par le fait que l'on retire de la partie d'épuisement de la colonne de distillation, en un courant latéral, les fractions d'acide acétique et d'alcool qui n'ont pas réagi.

5

Abb.